# EUROPÄISCHE PATENTSCHRIFT

(11) **EP 3 256 066 B1**
(45) Veröffentlichungstag und Bekanntmachung des Hinweises auf die Patenterteilung: **09.01.2019**
(21) Anmeldenummer: 16702360.5
(22) Anmeldetag: 25.01.2016
(51) Int. Cl.: A61B 18/24, A61B 17/00, A61B 18/22, A61B 18/00, A61B 34/20

(54) **LASERAPPLIKATOR MIT ELEKTRODEN**
LASER APPLICATOR HAVING ELECTRODES
APPLICATEUR LASER DOTÉ D'ÉLECTRODES

(30) Priorität: 09.02.2015 DE 102015202214
(43) Veröffentlichungstag der Anmeldung: 20.12.2017
(73) Patentinhaber: Vimecon GmbH, 52134 Herzogenrath (DE)
(72) Erfinder: MARKUS, Kai U., 52249 Eschweiler (DE); HETTKAMP, Martin, 52072 Aachen (DE)
(74) Vertreter: dompatent von Kreisler Selting Werner - Partnerschaft von Patent- und Rechtsanwälten mbB
(86) Internationale Anmeldenummer: PCT/EP2016/051421
(87) Internationale Veröffentlichungsnummer: WO 2016/128205

(56) Entgegenhaltungen:
- US-A1- 2011 264 000
- US-B1- 6 947 785

## Beschreibung

Die Erfindung betrifft einen Laserapplikator mit einem langgestreckten Katheter, der mindestens ein umfangsmäßig geschlossenes Lumen enthält und einem längs des Katheters verlaufenden Lichtleiter, der in einem distalen Endabschnitt des Katheters einen Auskoppelbereich aufweist.

Ein derartiger Laserapplikator ist beschrieben in WO 2007/118745 A1 (Vimecon), deren Inhalt durch Verweis in die vorliegende Beschreibung aufgenommen wird. Der bekannte Laserapplikator weist einen langgestreckten flexiblen Katheter auf, der einen Lichtleiter enthält. Der distale Endabschnitt ist zu einer lassoähnlichen Schleife geformt, deren Ebene quer zu dem Hauptteil des Katheters verläuft. Am proximalen Ende wird die Laserstrahlung in den Lichtleiter eingespeist. Am distalen Ende des Katheters befindet sich ein Auskoppelbereich, an dem die Energie seitlich aus dem Lichtleiter ausgekoppelt wird und den Katheter verlässt.

Das distale Ende ist das vordere, dem Patienten zugewandte und der Laserenergiequelle gegenüberliegende Ende des Katethers. Das proximale Ende ist das hintere, der Laserenergiequelle zugewandte und dem Patienten gegenüberliegende Ende des Katethers.

Der Laserapplikator dient insbesondere zur Behandlung von Vorhofflimmern und anderen Herz-Rhythmus-Störungen. Mit ihm kann Herzgewebe durch Umwandlung von Lichtenergie in thermische Energie verödet werden. Durch die aus dem Lichtleiter austretende Laserstrahlung wird das umgebende Gewebe soweit erhitzt, bis es zu einer Denaturierung von Proteinen und zum Ausbilden einer elektrisch inaktiven Narbe kommt.

In DE 10 2006 039 471 B3 ist ein Laserapplikator beschrieben, der einen Katheter mit einem Lichtleiter enthält. In einem distalen Endabschnitt des Katheters hat der Mantel des Lichtleiters eine Aussparung, an der Licht seitlich aus dem Lichtleiter austritt. Während der intakte Mantel des Lichtleiters für Totalreflexion sorgt, wodurch die Lichtenergie in Längsrichtung des Lichtleiters transportiert wird, bewirken die Aussparungen an der Grenze des Kernes des Lichtleiters eine Brechung, wodurch Strahlungsenergie ausgekoppelt wird.

In DE 10 2008 058 148 A1 (Vimecon), deren Inhalt ebenfalls durch Verweis in die vorliegende Beschreibung aufgenommen wird, ist ebenfalls ein Laserapplikator mit langestrecktem Katheter und längs des Katheters verlaufendem Lichtleiter mit einem Auskoppelbereich im distalen Endabschnitt des Katheters beschrieben.

US 6,947,785 B1 beschreibt einen Herzkatheter mit Elektroden zur diagnostischen Untersuchung von Herzgewebe.

US 2011/0264000 A1 beschreibt einen Herzkatheter mit Elektroden zur Bestimmung der Art von biologischem Gewebe und zur Erfassung der Gewebemorphologie.

Der Erfindung liegt die Aufgabe zugrunde, einen verbesserten Laserapplikator zu schaffen. Es soll insbesondere die Navigation des Katheters in umliegendem Gewebe erleichtert werden.

Der erfindungsgemäße Laserapplikator ist definiert durch Patentanspruch 1. Demnach ist der Laserabpplikator mit Elektroden versehen, die in Bezug auf den Auskoppelbereich einen definierten Abstand, sowie eine definierte Anordnung aufweisen.

Die Elektroden sind in der Lage, ein elektrisches Signal auszugeben, das von der Umgebung der Elektrode des Katheters bestimmt wird. Das elektrische Signal wird erfasst und ausgewertet. Anhand des elektrischen Signals kann auf die elektrische Leitfähigkeit des den Laserapplikator im Bereich der Elektrode umgebenden Materials und somit auf die Art des Materials geschlossen werden. Insbesondere kann unterschieden werden, ob die Elektrode von Luft, einer Flüssigkeit oder biologischem Gewebe kontaktiert bzw. umgeben wird. Weiterhin geben die elektrischen Signale Aufschluss über die Art des biologischen Gewebes. So kann beispielsweise zwischen Blut, Herzmuskel und Bindegewebe unterschieden werden.

Hierzu sind sämtliche der Elektroden elektrisch mit einer Auswerteeinrichtung verbunden, die die elektrischen Messsignale erfasst und auswertet. Dabei werden die Messwerte mit vorherigen Messwerten und/oder Referenzwerten verglichen. Die Referenzwerte können für bestimmte Materialien ausgewählt worden sein, wie z.B. ein erster Messwert für Luft, ein zweiter Messwert für Wasser oder eine Flüssigkeit und/oder ein dritter Messwert für biologisches Gewebe. Die Auswerteeinrichtung vergleicht den Messwert mit einem oder mehreren Referenzwerten und/oder zuvor aufgenommenen Messwerten und schließt anhand des Vergleichs auf das die Elektrode umgebende Material.

Der Auskoppelbereich des Laserapplikators erstreckt sich typischerweise über eine vorgegebene Länge in Längsrichtung des Katheters und über einen vorgegebenen Teilumfang in Umfangsrichtung des Katheters. Dadurch wird ein seitliches Abstrahlen von Laserenergie aus dem Laserapplikator ermöglicht. Wenn dabei mindestens eine Elektrode nur im Bereich eines Teilumfangs des Katheters eine nach außen hin freiliegende Kontaktfläche aufweist oder nach außen hin leitfähig ausgebildet ist, kann die relative Drehposition des Auskoppelbereichs in Bezug auf das von der Elektrode elektrisch kontaktierte Material bestimmt werden. Der von der Elektrode abgedeckte Teil des Katheters und der Abstand dieses Teilumfangs zu dem Auskoppelbereich müssen dabei jeweils bekannt sein.

In dem Auskoppelbereich sollte der Katheter keine Elektroden aufweisen, um ein Abdecken des Auskoppelbereichs und eine damit verbundene Beeinträchtigung der Lichtauskopplung zu vermeiden. Darüber hinaus würden typischerweise Metall aufweisende Elektroden im Auskoppelbereich von dem Laserlicht erhitzt werden, was vermieden werden sollte.

Vorteilhaft kann es sein, zwei Elektroden auf in Längsrichtung des Katheters einander gegenüberliegenden Seiten des Auskoppelbereichs anzuordnen, um die Position des Auskoppelbereichs in Längsrichtung des Katheters in Bezug auf den Katheter umgebendes Material erfassen zu können. Dabei kann der Auskoppelbereich, wie oben beschrieben, nur einen Teilumfang des Katheters abdecken. Alternativ kann diese Anordnung von Elektroden auch bei einem rundumabstrahlenden Auskoppelbereich zur Anwendung kommen.

Bei einem nur entlang eines Teilumfangs abstrahlenden Auskoppelbereich kann auf dem dem Auskoppelbereich gegenüberliegenden Umfangsbereich oder auf dem dem Auskoppelbereich identischen Umfangsbereich des Laserkatheters mindestens eine Elektrode angeordnet sein, die nur über einen Teilumfang nach außen hin leitfähig ausgebildet ist. Mit dieser Elektrode kann dann die relative Drehposition des Auskoppelbereichs in Bezug auf umgebendes Material ermittelt werden.

Die mindestens eine Elektrode kann eine freiliegende Kontaktfläche aufweisen, die sich je nach Anwendungsfall über den gesamten Umfang des Katheters oder nur über einen Teilumfang des Katheters erstreckt. Denkbar ist auch eine umfangsmäßig umlaufende Elektrode (Ringelektrode), die zu einem Teil des Umfangs mit einem Isolator bedeckt ist, so dass nur der verbleibende, nicht von dem Isolator bedeckte Teilumfang nach außen hin leitfähig ist.

Zwei derartige, nur über einen Teilumfang leitfähig ausgebildete Elektroden können in einem Abstand zueinander angeordnet sein, um die Torsion des Katheters zwischen den beiden Elektroden bestimmen zu können. Der relative Drehwinkel zwischen den Elektroden muss dabei bekannt sein.

Mit der Erfindung wird erstmals ermöglicht, die Position eines Laserapplikators in Bezug auf umgebendes Gewebe zu erfassen, ohne dass separate Hilfsmittel eingesetzt werden. Dies ist insbesondere von Bedeutung, da der Laserkatheter im Anwendungsfall typischerweise nur durch eine kleine Körperöffnung ("Schlüsselloch") in den Patienten eingeführt wird. Dem Arzt stehen die Messsignale der Elektroden als Hilfsmittel zu Verfügung, um den Auskoppelbereich in ausreichende Deckung mit dem zu abladierenden Gewebe bringen zu können.

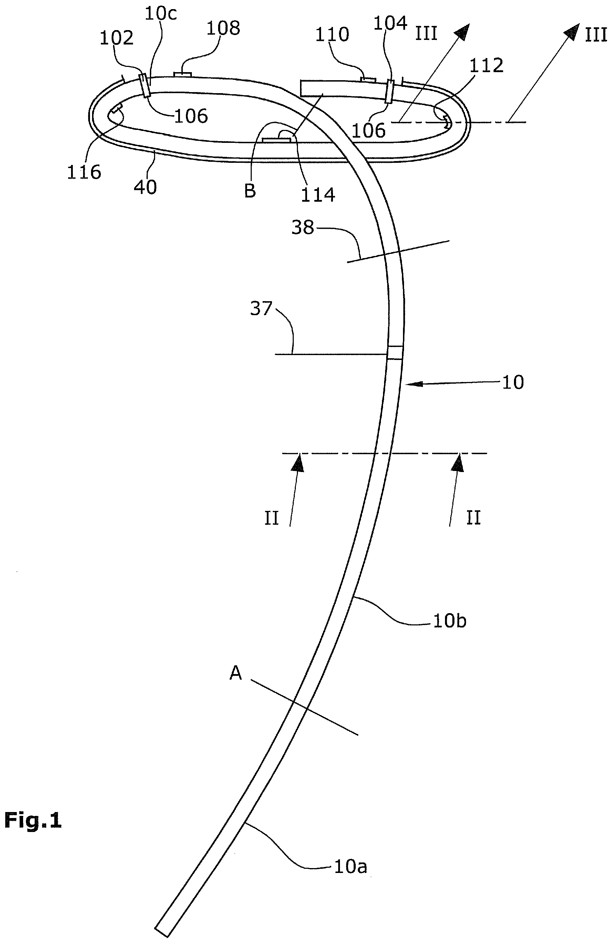

Im Folgenden wird anhand der Figuren ein Ausführungsbeispiel der Erfindung näher erläutert. Es zeigen:
- Fig. 1: eine schematische Darstellung der generellen Struktur des Lichtleiters,

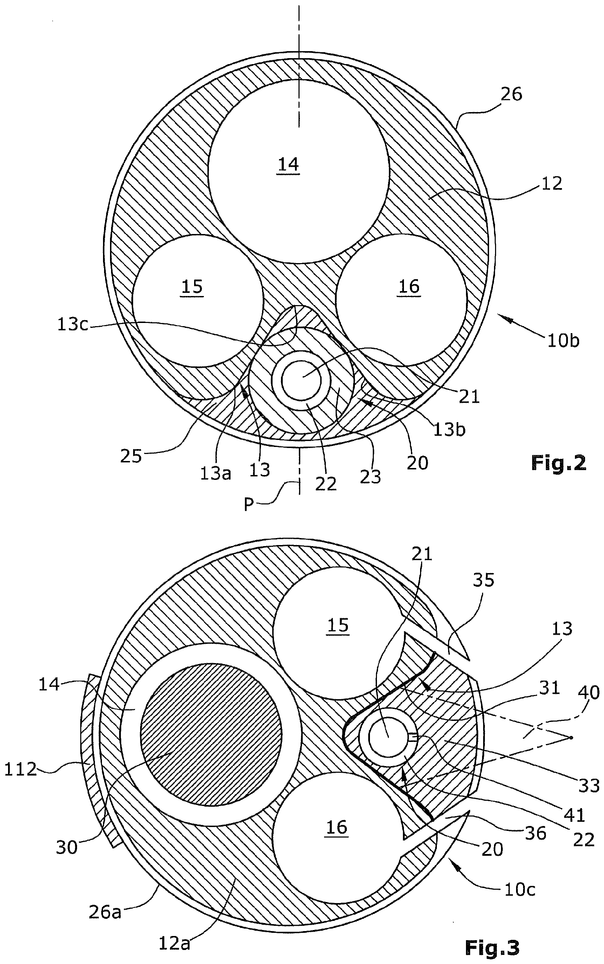

- Fig. 2: einen Querschnitt entlang der Linie II-II im Mittelabschnitt des Katheters gemäß Fig. 1,
- Fig. 3: einen Querschnitt entlang der Linie III-III im distalen Endabschnitt des Katheters gemäß Fig. 1,
- Fig.4: eine perspektivische Ansicht eines weiteren Ausführungsbeispiels und
- Fig. 5: eine Draufsicht auf das weitere Ausführungsbeispiel aus Richtung des Pfeils V in Figur 4.
Der in den Figuren dargestellte Laserapplikator ist in dessen überwiegenden Details in DE 10 2008 058 148 A1 beschrieben. Hierbei sind in den Figuren folgende Elemente mit Bezugszeichen versehen:
- 10: Katheter
- 10a: Abschnitt
- 10b: Mittelabschnitt, Abschnitte
- 10c: Endabschnitt
- 12: Katheterkörper
- 12a: Katheterkörper
- 13: Nut
- 13a: Flanken
- 13b: Flanken
- 13c: Basis
- 14: Lumen
- 15: Kühlkanäle
- 16: Kühlkanäle
- 20: Lichtleiter
- 21: Kern
- 22: Mantel
- 23: Schutzhülle
- 25: Kleber
- 26: Umschlauch
- 26a: Umschlauch
- 30: Formungsdraht
- 31: Reflexionsschicht
- 33: Material
- 35: Austrittsbohrungen
- 36: Austrittsbohrungen
- 37: Katheterspleißstelle
- 38: Schlauchspleißstelle
- 40: Auskoppelbereich
- 41: Öffnungen
Im Folgenden wird die über DE 10 2008 058 148 A1 hinausgehende Offenbarung erläutert:
Die Länge des Auskoppelbereichs in Längsrichtung des Katheters ist in Fig. 1 mit dem Bezugszeichen 40 gekennzeichnet. Proximal des Auskoppelbereichs 40 und distal des Auskoppelbereichs 40 sind jeweils eine Ringelektrode 102, 104 angeordnet. Die Ringelektroden 102, 104 zeichnen sich dadurch aus, dass sie entlang des Umfangs des Laserapplikators vollständig umlaufend elektrisch leitfähig mit einer elektrischen Kontaktfläche 106 ausgebildet sind. Mit Hilfe der Messsignale der beiden Ringelektroden 102, 104 kann die Position des Auskoppelbereichs 40 in Längsrichtung des Katheters in Bezug auf von den Elektroden 102, 104 kontaktiertes Gewebe ermittelt werden. Weiterhin sind diese Ringelelektroden in der Röntgendurchleuchtung sichtbar und kennzeichnen den Anfang und das Ende des Auskoppelbereichs 40.

Darüber hinaus sind proximal und distal des Auskoppelbereichs 40 jeweils eine Punktelektrode 108, 110 angeordnet. In Umfangsrichtung des Katheters liegen die Kontaktflächen der beiden Punktelektroden 108, 110 auf gleichen Positionen, wenn der Katheter nicht verdreht ist. Der relative Winkel zwischen den beiden Radialen vom Mittelpunkt des Katheters durch den Mittelpunkt der jeweiligen Elektrode 108, 110 ist dann 0 Grad. Sobald sich der Katheter durch Torsion verdreht, verändert sich der relative Drehwinkel zwischen den beiden Elektroden 108, 110. Wenn dieser Drehwinkel von 0 abweicht, unterliegt der Katheter einer Torsion. Der Grad der Torsion des Katheters kann mit Hilfe der Punktelektroden 108, 110 erfasst werden.

Mit dem Begriff "Punktelektrode" wird vorliegend allgemein eine Einzelelektrode bezeichnet, wobei der Begriff "Punkt" nicht im mathematischen Sinne zu verstehen ist. Vielmehr ist mit "Punktelektrode" eine Elektrode gemeint, die das Signal an einem einzelnen Punkt oder eines eng eingegrenzten Bereichs des Gewebes aufnehmen soll. Die Punktelektrode kann kreis- beziehungsweise scheibenförmig ausgebildet sein.

Auf der dem Auskoppelbereich 40 gegenüberliegenden Seite des Katheters sind weitere Elektroden 112, 114, 116 angeordnet, die jeweils nur einen Teilumfang des Katheters elektrisch leitfähig überdecken. Mit Hilfe dieser Elektroden 112, 114, 116 kann der relative Drehwinkel des Auskoppelbereichs 40 in Bezug auf von den Elektroden 112, 114, 116 kontaktiertes Gewebe ermittelt werden. Insbesondere dienen diese Elektroden 112, 114, 116 der Bestimmung des Kontaktes des Auskoppelbereiches 40 mit Gewebe, wie zum Beispiel Herzmuskelgewebe. Denn solange der Auskoppelbereich Kontakt zu Gewebe hat, dürften die Elektroden auf der Rückseite des Auskoppelbereiches keinen Kontakt haben.

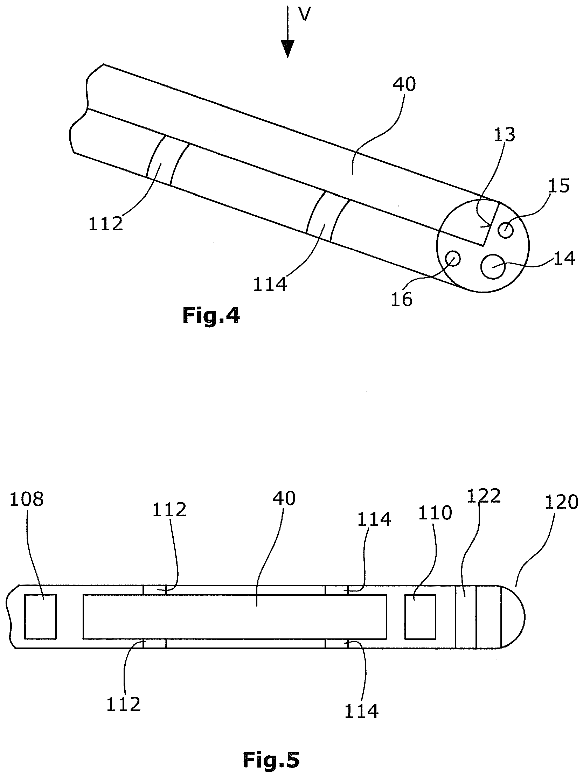

Bei dem Ausführungsbeispiel gemäß den Figuren 4 und 5 sind auf der dem Auskoppelbereich 40 gegenüberliegenden Seite des Katheters zwei Elektroden 112, 114 beabstandet zueinander angeordnet. Diese Elektroden sind jeweils nach Art von Ringelektroden ausgebildet, die den nicht von dem Auskoppelbereich 40 abgedeckten Umfang abdecken. Der Umfangsanteil des Auskoppelbereichs 40 ist also bei jeder der beiden Elektroden 112, 114 ausgespart. Wenn eine der Elektroden 112, 114 ein elektrisches Signal aufgrund von Kontakt mit Gewebe aussendet, kann dies als Hinweis darauf gesehen werden, dass der Auskoppelbereich 40 im Bereich der betreffenden Elektrode keinen Gewebekontakt hat.

Weiterhin ist eine Elektrode 108 proximal des Auskoppelbereichs 40 und eine weitere Elektrode 110 distal des Auskoppelbereichs 40 auf derselben Seite des Katheters wie der Auskoppelbereich 40 angeordnet. Die beiden Elektroden 108, 110 decken dabei jeweils denselben Umfangsabschnitt des Katheters wie der Auskoppelbereich 40 ab. Wenn also beide Elektroden 108, 110 ein durch Kontakt mit Gewebe hervorgerufenes elektrisches Signal aussenden, kann dies als Hinweis darauf gesehen werden, dass der dazwischen liegende Auskoppelbereich 40 ebenfalls Kontakt mit Gewebe hat.

Am distalen Ende des Katheters ist bei dem Ausführungsbeispiel gemäß Figur 5 eine distale Endelektrode 120 als Vollelektrode ausgebildet. Das bedeutet, dass die Elektrode 120 die distale Stirnfläche des Katheters vollständig abdeckt. Die Elektrode 120 des Ausführungsbeispiels ist kugelkappenförmig ausgebildet, um ein stumpfes Ende des Katheters zu bilden.

Proximal der Vollelektrode 120 ist eine Ringelektrode 122 in einem geringen Abstand von wenigen Millimetern (weniger als 1cm) angeordnet. Die Ringelektrode 120 ist eine den vollständigen Umfang des Katheters abdeckende Vollelektrode.

## Patentansprüche

1. Laserapplikator mit einem langgestreckten Katheter (10), der mindestens ein umfangsmäßig geschlossenes Lumen (14) enthält und einen längs des Katheters verlaufenden Lichtleiter (20), der in einem distalen Endabschnitt (10c) des Katheters einen Auskoppelbereich (40) aufweist,
wobei der Laserapplikator mindestens eine Elektrode (102, 104, 108, 110, 112, 114, 116) in einem zu dem Auskoppelbereich definierten Abstand aufweist, um die Position des Auskoppelbereichs (40) in Bezug auf umgebendes Gewebe erfassen zu können, und
wobei die Elektrode eine nach außen hin freiliegende Kontaktfläche (106) aufweist,
**dadurch gekennzeichnet,**
**dass** die Elektrode (108, 110, 112, 114, 116) nur im Bereich eines Teilumfangs des Katheters eine nach außen hin freiliegende Kontaktfläche (106) aufweist, um die relative Drehposition des Auskoppelbereichs gegenüber kontaktiertem Gewebe bestimmen zu können, wobei sich der Auskoppelbereich (40) in Umfangsrichtung des Katheters ebenfalls nur über einen Teilumfang erstreckt.

2. Laserapplikator nach Anspruch 1, **dadurch gekennzeichnet, dass** die Elektrode im Bereich des distalen Endabschnitts (10c) angeordnet ist.

3. Laserapplikator nach Anspruch 1 oder 2, **dadurch gekennzeichnet, dass** mindestens eine Elektrode (104, 110) distal des Auskoppelbereichs (40) angeordnet ist.

4. Laserapplikator nach einem der Ansprüche 1 bis 3, **dadurch gekennzeichnet, dass** zwei Elektroden (102, 104) auf in Längsrichtung des Katheters einander gegenüberliegenden Seiten des Auskoppelbereichs (40) angeordnet sind, um die Position des Auskoppelbereichs in Längsrichtung des Katheters in Bezug auf umgebendes Gewebe erfassen zu können.

5. Laserapplikator nach einem der einem der Ansprüche 1 bis 4, **dadurch gekennzeichnet, dass** auf der dem Auskoppelbereich (40) gegenüberliegenden Seite des Laserkatheters mindestens eine Elektrode (114, 116) angeordnet ist, die nur über einen Teilumfang des Katheters nach außen hin leitfähig ausgebildet ist.

6. Laserapplikator nach einem der Ansprüche 1 bis 5, **dadurch gekennzeichnet, dass** mindestens eine Elektrode in Umfangsrichtung des Katheters umlaufend ausgebildet ist und zu einem Teil des Umfangs mit einem Isolator nach außen hin bedeckt ist.

7. Laserapplikator nach Anspruch 6, **dadurch gekennzeichnet, dass** mindestens zwei in Umfangsrichtung umlaufend ausgebildete und zu einem Teil des Umfangs nach außen mit einem Isolator bedeckte Elektroden in einem Abstand zueinander angeordnet sind, um die Torsion des Katheters zwischen den beiden Elektroden bestimmen zu können.

8. Laserapplikator nach einem der Ansprüche 1 bis 7, **dadurch gekennzeichnet, dass** eine elektrisch mit jeder Elektrode verbundene Auswertevorrichtung zum Empfangen und Auswerten der Elektrodensignale vorgesehen ist.

9. Laserapplikator nach einem der vorhergehenden Ansprüche, **dadurch gekennzeichnet, dass** proximal und/oder distal des Auskoppelbereichs (40) jeweils eine Elektrode (108, 110) auf derselben Seite des Katheters wie der Auskoppelbereich (40) angeordnet ist.

10. Laserapplikator nach Anspruch 9, **dadurch gekennzeichnet, dass** die proximal und distal des Auskoppelbereichs (40) angeordneten Elektroden (108, 110) denselben Umfang des Katheters wie der Auskoppelbereich (40) abdecken.

11. Laserapplikator nach einem der vorhergehenden Ansprüche, **dadurch gekennzeichnet, dass** am distalen Ende des Katheters eine vollständig umlaufende Ringelektrode (122) und/oder eine die distale Stirnfläche des Katheters bedeckende Elektrode (120) vorgesehen ist.

## Claims

1. Laser applicator comprising an elongate catheter (10) which contains at least one peripherally closed lumen (14), and a light guide (20), which extends along the catheter and has an outcoupling region (40) in a distal end section (10c) of the catheter,
wherein said laser applicator has at least one electrode (102, 104, 108, 110, 112, 114, 116) at a distance defined in relation to the outcoupling region such that the position of the outcoupling region (40) in relation to surrounding tissue can be sensed, and
wherein the electrode has a contact surface (106) exposed to the outside,
**characterized in that**
the electrode (108, 110, 112, 114, 116) has a contact surface (106) exposed to the outside only in the region of a partial circumference, so as to allow the determination of the relative position of rotation of the outcoupling region with respect to contacted tissue, the outcoupling region (40) also extending only over a partial circumference in the circumferential direction of the catheter.

2. Laser applicator of claim 1, **characterized in that** the electrode is arranged in the region of the distal end section (10c).

3. Laser applicator of claim 1 or 2, **characterized in that** at least one electrode (104, 110) is arranged distally of the outcoupling region (40).

4. Laser applicator of one of claims 1 to 3, **characterized in that** two electrodes (102, 104) are arranged on sides of the outcoupling region (40) opposing each other in the longitudinal direction of the catheter, so as to be able to sense the position of the outcoupling region in the longitudinal direction of the catheter with respect to surrounding tissue.

5. Laser applicator of one of claims 1 to 4, **characterized in that** on the side of the laser catheter opposite the outcoupling portion (40), at least one electrode (114, 116) is arranged which is conductive to the outside only over a partial circumference of the catheter.

6. Laser applicator of one of claims 1 to 5, **characterized in that** at least one electrode is formed to extend circumferentially in the circumferential direction of the catheter and, for a part of the circumference, is covered with an insulator towards the outside.

7. Laser applicator of claim 6, **characterized in that** at least two electrodes, which are formed to extend circumferentially in the circumferential direction and covered with an insulator towards the outside for a part of the circumference, are arranged at a distance from each other so as to allow the determination of the torsion of the catheter between the two electrodes.

8. Laser applicator of one of claims 1 to 7, **characterized in that** an evaluation means is provided for receiving and evaluating the electric signal, the evaluation means being electrically connected with each electrode.

9. Laser applicator of one of the preceding claims, **characterized in that** proximally and/or distally of the outcoupling region (40) an electrode (108, 110) is respective arranged on the same side of the catheter as the outcoupling region (40).

10. Laser applicator of claim 9, **characterized in that** the electrodes (108, 110) arranged proximally and distally of the outcoupling region (40) cover the same circumference as the outcoupling region (40).

11. Laser applicator of one of the preceding claims, **characterized in that** a fully circumferentially extending annular electrode (122) and/or an electrode (120) covering the distal end face of the catheter are arranged at the distal end of the catheter.

## Revendications

1. Applicateur laser avec un cathéter (10) allongé qui contient au moins une lumière (14) fermée sur la circonférence et, le long du cathéter, un guide de lumière (20) qui, dans un tronçon d'extrémité (10c) distal du cathéter, comporte une zone de découplage (40),
l'applicateur laser comportant au moins une électrode (102, 104, 108, 110, 112, 114, 116) à distance définie de la zone de découplage pour pouvoir détecter la position de la zone de découplage (40) par rapport à un tissu environnant, et
l'électrode comportant une zone de contact (106) exposée vers l'extérieur,
**caractérisé en ce que**
l'électrode (108, 110, 112, 114, 116) ne comporte une zone de contact (106) exposée vers l'extérieur que dans la zone d'une circonférence partielle du cathéter pour pouvoir définir la position de rotation relative de la zone de découplage vis-à-vis du tissu contacté, la zone de découplage (40) s'étendant dans la direction circonférentielle du cathéter également uniquement sur une circonférence partielle.

2. Applicateur laser selon la revendication 1, **caractérisé en ce que** l'électrode est disposée dans la zone du tronçon d'extrémité (10c) distal.

3. Applicateur laser selon la revendication 1 ou 2, **caractérisé en ce qu'**au moins une électrode (104, 110) est disposée de façon distale à la zone de découplage (40) .

4. Applicateur laser selon l'une des revendications 1 à 3, **caractérisé en ce que** deux électrodes (102, 104) sont disposées sur des côtés de la zone de découplage (40) opposés dans la direction longitudinale du cathéter pour pouvoir détecter la position de la zone de découplage dans la direction longitudinale du cathéter par rapport à un tissu environnant.

5. Applicateur laser selon l'une des revendications 1 à 4, **caractérisé en ce que**, sur le côté du cathéter laser opposé à la zone de découplage (40), il est disposé au moins une électrode (114, 116) qui n'est constituée de façon conductrice vers l'extérieur que sur une circonférence partielle du cathéter.

6. Applicateur laser selon l'une des revendications 1 à 5, **caractérisé en ce qu'**au moins une électrode est constituée de façon circulaire dans la direction circonférentielle du cathéter et est couverte par un isolateur vers l'extérieur sur une partie de la circonférence.

7. Applicateur laser selon la revendication 6, **caractérisé en ce qu'**au moins deux électrodes constituées de façon circulaire dans la direction circonférentielle et couvertes sur une partie de la circonférence vers l'extérieur avec un isolateur sont disposées à distance l'une de l'autre pour pouvoir définir la torsion du cathéter entre les deux électrodes.

8. Applicateur laser selon l'une des revendications 1 à 7, **caractérisé en ce qu'**un dispositif d'analyse raccordé électriquement à chaque électrode est prévu pour la réception et l'analyse des signaux d'électrode.

9. Applicateur laser selon l'une des revendications précédentes, **caractérisé en ce que**, de façon proximale et/ou distale à la zone de découplage (40), respectivement une électrode (108, 110) est disposée sur le même côté du cathéter que la zone de découplage (40).

10. Applicateur laser selon la revendication 9, **caractérisé en ce que** les électrodes (108, 110) disposées de façon proximale et/ou distale à la zone de découplage (40) recouvrent la même circonférence du cathéter que la zone de découplage (40).

11. Applicateur laser selon l'une des revendications précédentes, **caractérisé en ce que**, à l'extrémité distale du cathéter, il est prévu une électrode annulaire (122) entièrement circulaire et/ou une électrode (120) couvrant la face frontale distale du cathéter.
